# EUROPÄISCHE PATENTANMELDUNG

(11) **EP 0 999 266 A1**
(43) Veröffentlichungstag der Anmeldung: **10.05.2000**
(21) Anmeldenummer: 98120337.5
(22) Anmeldetag: 27.10.1998
(51) Int. Cl.: C12M 1/22

(54) **Verfahren und Vorrichtung zur Aufnahme einer Zellkultur**

(30) Priorität: 24.09.1998 DE 29817223 U
(71) Anmelder: Marotzki, Stefan, Dr., 25436 Tornesch (DE)
(72) Erfinder: Marotzki, Stefan, Dr., 25436 Tornesch (DE)
(74) Vertreter: Glawe, Delfs, Moll & Partner

(57) **Zusammenfassung**

Vorrichtung zur Aufnahme einer Zellkultur mit einem Gefäß (1), das einen Boden (3) und Wände (4) aufweist, sowie mit einem Deckel (2). Dieser ist in das Gefäß (1) einführbar. Zur Abfuhr verdrängter Luft und Überschußflüssigkeit ist er mit einer Abströmöffnung (6) versehen. Der Deckel (2) kann in unterschiedlicher Weise dicht an die Gefäßwände (4) angeschlossen werden. Die Abströmöffnung kann nach dem Einsetzen des Deckels geschlossen werden. Innerhalb des Gefäßes (1) können durch am Deckel (2) vorgesehene Einrichtungen (31) voneinander gesonderte Kammern (30) gebildet werden.

## Beschreibung

Für die molekularbiologische oder gentechnische Behandlung oder Untersuchung von Zellen verwendet man Vorrichtungen, die einerseits aus einem Gefäß (beispielsweise Petrischale) zur Aufnahme der Zellen und einer Reaktionsflüssigkeit und andererseits aus einem Deckel bestehen (Prospekt "Lab-Tek II" der Nalge Nunc International, Naperville; Prospekt "EasiSeal" der HYBAID Limited, Teddigton; Prospekt "Gene Frame" der Advanced Biotechnologies Ltd., Epsom; EP-A 611 598). Dabei sind Deckel zu unterscheiden, die lediglich lose auf den oberen Gefäßrand aufgelegt werden, und luftdichte Abdeckungen, die mit dem Rand der Vorrichtung oder einem den Boden der Vorrichtung bildenden Objektträger adhäsiv und luftdicht verbunden werden. Beim Auflegen der letzteren muß man darauf achten, daß Luftblasen zwischen der Abdeckung und dem Substrat vermieden werden. Dies geschieht in der Regel dadurch, daß ein Teil der Reaktionsflüssigkeit beim Auflegen der Abdeckung zur Seite hin verdrängt wird. Dies ist aber nur dann möglich, wenn das Gefäß keine Wände aufweist. Da diese in bestimmten Verfahrensstufen erwünscht sind, sieht man dicht mit dem Boden (Objektträger) der Vorrichtung verbindbare und davon lösbare Wände vor. Die Entfernbarkeit der Wände ist bei den bekannten Vorrichtungen auch deshalb erforderlich, weil die Substratschicht dünn sein soll, damit die während des Verfahrens notwendigen Temperaturwechsel rasch und genau steuerbar vollzogen werden können.

Der Erfindung liegt die Aufgabe zugrunde, ein Verfahren bzw. eine Vorrichtung zu schaffen, die den Abschluß des ggf. dünnschichtigen Substrats auch dann gestattet, wenn Wände vorhanden sind. Die Lösung besteht in den Merkmalen der Ansprüche 1 oder 2.

Beim Einführen des Deckels zwischen die Wände des Gefäßes wird die oberhalb des Substrats im Gefäß befindliche Luft durch die Abströmöffnung verdrängt. Auch ein möglicher Überschuß an Reaktionsflüssigkeit wird durch die Abströmöffnung verdrängt. Der Deckel kann soweit innerhalb des Gefäßes abgesenkt werden, bis zwischen der Bodenplatte und der dieser gegenüberliegenden Deckelplatte des Deckels die gewünschte Dicke der Substratschicht erreicht ist, die von wenigen Hundertstel Millimetern bis zu einigen Millimetern reichen kann.

Zur Vermeidung unerwünschten Totraums kann die Umfangstläche des Deckels sich im wesentlichen formgleich und eng an die Innenfläche der Wände anschließen. Dieser Anschluß kann auch die Abdichtung des Deckels gegenüber den Wänden beinhalten, beispielsweise durch Glasschliff oder unter elastischem Druck anliegende Kunststoffdichtung. Jedoch muß dies nicht der Fall sein, da zusätzlich zu einem engen, aber nicht dichten Anschluß der Umfangsfläche des Deckels an die Innenfläche der Wände eine besondere Dichtung vorgesehen sein kann, die beispielsweise mit dem Rand der Gefäßwände oder einem Absatz derselben zusammenwirken kann. In diesen Fällen ist es oft zweckmäßig, eine Halterung zur Aufrechterhaltung der Dichtposition des Deckels gegenüber dem Gefäß vorzusehen, beispielsweise einen Schraub-, Schnapp- oder Federverschluß; jedoch kann der dichte Anschluß des Deckels an die Gefäßwandung auch selbsthaltend sein, beispielsweise durch die Reibkräfte, die zwischen zwei Glasschliffflächen bestehen.

Zweckmäßig ist es, wenn die unterseitige Deckelplatte eine etwa parallel zum Boden des Gefäßes verlaufende Unterfläche aufweist, um eine im wesentlichen konstante Schichtdicke bewirken zu können. Der Deckel und das Gefäß können mit zusammenwirkenden Anschlagflächen versehen sein, die die Größe und Konstanz der Schichtdicke bestimmen.

Die Abströmöffnung kann mit einer Verschlußvorrichtung versehen sein. Jedoch besteht auch die Möglichkeit der Abdichtung mittels einer nachträglich aufgebrachten Ölschicht. Dies gilt auch für die Umfangsdichtung. In vielen Fällen ist dichter Abschluß lediglich gegenüber der äußeren Atmosphäre notwendig, um beispielsweise Verdunstung oder den Zutritt von Sauerstoff zu vermeiden. In diesen Fällen kann es genügen, wenn die Abdichtung nicht am Gefäß und Deckel, sondern von einer die Vorrichtung aufnehmenden Apparatur gebildet wird, beispielsweise einer Art Autoklaven, in welchem eine innere Atmosphäre herrscht, die nach den gewünschten Zwecken gewählt ist. Zur vermeidung von Verdunstung kann sie beispielsweise hinreichende Feuchte aufweisen. Zur Vermeidung von Zutritt schädlicher Gase kann sie aus Stickstoff oder Edelgas bestehen.

Damit nicht nur die Luft im Bereich der Abströmöffnung entfernt wird, sondern auch derjenige Luftanteil, der zwischen der Umfangsfläche des Deckels und den Gefäßwänden enthalten sein mag, kann vorgesehen sein, daß die Abströmöffnung mit einer Steigstrecke versehen ist, deren Mündungshöhe mindestens etwa ebenso hoch ist wie das obere Ende des zwischen der Umfangsfläche des Deckels und den Gefäßwänden befindlichen Spalts. Bei langsamem Einsetzen des Deckels in das Gefäß sorgt dann der statische Druck der in der Steigstrecke der Abströmöffnung befindlichen Flüssigkeitssäule dafür, daß auch die Luft aus dem Umfangsspalt verdrängt wird.

Wenn die Zellen hinreichend fest dem Gefäßboden angelagert sind, kann die Überschußflüssigkeit ohne weiteres durch die Abströmöffnung hindurch abgezogen bzw. verdrängt werden. Damit auch mit Zellen gearbeitet werden kann, die sich in Suspension befinden, kann die Abströmöffnung mit einem Sieb versehen werden, das die Zellen bei der Verdrängung der Flüssigkeit zurückhält.

Es können mehrere Öffnungen vorgesehen sein, die zum Anschluß einer Mediumszu- und -ableitung ausgestaltet sind. Eine oder mehrere dieser Öffnungen können als Abströmöffnung dienen. Die Vorrichtung kann dann auch als sogenannter Reaktor (Meenen et al.; "Semi Continuous Reactor System ...", Poster 1994 Biomaterials 21:905-908) verwendet werden.

Bei den aufgenommenen Zellen kann es sich um adhärente Zellen handeln, die am Boden der Zellkulturschale haften, oder um Suspensionszellen, die im Kulturmedium schwimmen. Alternativ können in der Schale auch Gewebeschnitte kultiviert werden. Allgemein können sowohl eukaryotische als auch prokaryotische Zellen in dem Gefäß kultiviert werden. Behandlungen und Untersuchungen, die mit der erfindungsgemäßen Vorrichtung durchzuführen sind, können beispielsweise alle Arten der in situ Hybridisierung und in situ PCR sein.

Die Abströmöffnung wird zweckmäßigerweise innerhalb des Deckeis in Abstand von dessen Rand vorgesehen. Jedoch soll nicht ausgeschlossen werden, daß sie durch einen mindestens stellenweise vorgesehenen Abstand zwischen dem Deckelrand und den Gefäßwänden gebildet ist. Besonders vorteilhaft ist in diesem Zusammenhang eine Ausführung, bei der der Deckelrand einen von der unterseitigen Deckelplatte hochstehenden Kragen umfaßt, der in seiner Gesamtheit oder stellenweise den genannten Abstand zur Bildung einer Abströmöffnung bildet und dessen oberer Rand mit den Gefäßwänden zur Bildung einer Dichtung zusammenwirkt. Während des Einsenkens des Deckels können dann zwischen Deckel und Gefäß eingeschlossene Gas- und Flüssigkeitsanteile abströmen, und erst am Schluß dieses Vorgangs, wenn der Deckel seine Endposition erreicht, kommt die Dichtung zustande.

Wenn man mehrere Kulturen denselben thermischen Bedingungen unterwerfen will, ist es zweckmäßig, mehrere Gefäße miteinander zu verbinden. Dabei kann für jedes einzelne der Gefäße ein Deckel der oben bezeichneten Art vorgesehen sein. Jedoch ist es auch möglich, mehrere Deckel zu gemeinsamer Betätigung miteinander zu verbinden. Zwar ist es möglich, die Wände mehrerer Gefäße einstückig miteinander auszuführen, wobei entweder die Böden auch einstückig mit den Wänden verbunden sind oder von diesen, beispielsweise in der Form eines Objektträgers, trennbar sind.

Von besonderer Bedeutung ist im Zusammenhang der Erfindung aber die Möglichkeit, durch Wandbildung innerhalb eines und desselben Gefäßes mehrere Kammern schaffen zu können, die im Laufe der Behandlung einer Kultur je nach Wunsch zeitweise voneinander getrennt oder nicht getrennt sind. Zu diesem Zweck wird der Deckel mit einer Einrichtung zum Abteilen solcher Kammern voneinander versehen. Besonders vorteilhaft ist eine Ausführung, bei welcher ein engeres Gefäß so ausgebildet ist, daß es in einem weiteren Gefäß untergebracht werden kann. Der Deckel des weiteren Gefäßes ist dabei in der Regel so ausgebildet, daß er lediglich zum Verschluß des außerhalb des engeren Gefäßes befindlichen Bereichs des weiteren Gefäßes dient. Für den Verschluß des engeren Gefäßes ist dann gegebenenfalls ein besonderer Verschluß vorgesehen, für den auch das gilt, was weiter oben allgemein für den Verschluß der Gefäße angegeben wurde. Das heißt, außer einem einfachen luftdurchlässigen oder luftdichten Verschluß kann auch ein Deckel vorgesehen werden, der mit einer Öffnung zur Verdrängung der im engeren Gefäß anstehenden Atmosphäre und gegebenenfalls Überschußflüssigkeit eingerichtet ist und dessen Luftöffnung verschließbar ist. Es besteht aber auch die Möglichkeit, die Deckel des weiteren und des engeren Gefäßes konstruktiv zu gemeinsamer Betätigung miteinander zu verbinden.

Wenn in vorliegendem Zusammenhang von "einem" engeren Gefäß die Rede ist, so soll dies heißen, daß dies wenigstens eins ist; es können auch mehrere engere Gefäße in einem weiteren Gefäß vorgesehen sein. Die Anordnung eines engeren Gefäßes in einem weiteren Gefäß gibt die Möglichkeit, die Kulturen innerhalb des engeren und des weiteren Gefäßes genau denselben thermischen Bedingungen zu unterwerfen. Sie können gesonderte Böden haben. Von besonderer Bedeutung ist aber eine Ausführung, bei welcher das engere Gefäß den Boden mit dem weiteren Gefäß teilt. Dies gibt die Möglichkeit, eine und dieselbe, innerhalb des weiteren Gefäßes angelegte Kultur während aller oder eines Teils der Reaktionsschritte unterschiedlichen Reaktionsbedingungen (beispielsweise einer anderen Reaktionsflüssigkeit) zu unterwerfen. Dementsprechend wird die Wand des engeren Gefäßes lediglich in denjenigen Reaktionsschritten innerhalb der sonst einheitlichen Kultur eingesetzt, in denen die Reaktionsbedingungen unterschiedlich sein sollen, z. B. Positivkontrolle oder Negativkontrolle der PCR.

Es gibt Fälle, in denen das engere Gefäß während der gesamten Reaktion bzw. Reaktionsfolge in dem weiteren Gefäß verbleiben kann. In diesen Fällen kann es von vornherein fabrikmäßig mit dem Boden und dem weiteren Gefäß bleibend verbunden werden. In anderen Fällen wird die Kompartimentierung zu Beginn der Reaktion oder Reaktionskette gewünscht; dann kann das engere Gefäß zwar ebenfalls fabrikmäßig mit dem Boden des weiteren Gefäßes verbunden sein, ist aber lösbar, damit die Kompartimentierung im gewünschten Zeitpunkt aufgehoben werden kann. Die fabrikmäßige Anordnung hat den Vorteil, daß das engere Gefäß im Verhältnis zu den Wänden des weiteren genau positioniert werden kann und daher Gewähr dafür gegeben ist, daß der Deckel paßt. In wieder anderen Fällen will man Freiheit haben, die Kompartimentierung in einem beliebigen Zeitpunkt vornehmen zu können. In diesen Fällen ist die Wand des engeren Gefäßes vom Benutzer einsetzbar.

Wenn der Deckel das weitere Gefäß vollständig abschließen soll, muß er nicht nur mit seiner Umfangsfläche der Wand des weiteren Gefäßes angepaßt sein, sondern auch hinreichend dicht mit dem engeren Gefäß verbunden werden können. Dies kann dadurch geschehen, daß er einen der Form des engeren Gefäßes angepaßten Ausschnitt aufweist, der mit der gewünschten Dichtheit daran anschließen kann. Dieser Ausschnitt kann von einer Abströmöffnung gebildet sein. In einer anderen Ausführungsform ist der Deckel mit der Wandung des engeren Gefäßes einstückig verbunden, so daß die durch das engere Gefäß ermöglichte Kompartimentierung mit dem Einsetzen des Deckels zustande kommt. In beiden Fällen dient der Deckel als Halterung oder Führung beim Einsetzen und gegebenenfalls auch bei der Benutzung des engeren Gefäßes.

In manchen Fällen ist ein absolut dichter Abschluß der Wand des engeren Gefäßes gegenüber dem Boden des weiteren Gefäßes nicht erforderlich; das engere Gefäß kann dann einfach von einem Röhrchen gebildet sein, das vom Deckel gehalten wird und bis auf den Boden des weiteren Gefäßes hinabreicht. Wenn dichter Anschluß der engeren Gefäßwand an den Boden verlangt wird, kann der Unterrand der Wand des engeren Gefäßes mit einer Dichtung versehen sein, wobei die Wand zur Erzeugung der Dichtpressung durch den Deckel oder eine zusätzliche Klammer gegen den Boden gepreßt wird. Die Wand des engeren Gefäßes kann auch mit dem Boden verklebt werden. Im einfachsten Fall besteht die Gefäßwand des engeren Gefäßes aus einem Dichtungsring, der zwischen der Bodenplatte und der Deckelplatte eingelegt ist, an diese dicht anschließt und die von ihm eingeschlossene Kammer von dem umliegenden Raum abteilt. Damit der eingeschlossenen Kammer eine unterschiedliche Behandlung zuteil werden kann, muß sie durch eine in ihrem Bereich befindliche Deckelöffnung zugänglich sein, bei der es sich um die Abströmöffnung handeln kann.

Der Deckel kann nach der Erfindung auch mit weiteren Einrichtungen versehen sein, die für die Behandlung der Kulturen gewünscht sind. Für die Elektroporation können der Deckel und der Boden beispielsweise flächig ausgebildete Elektroden tragen und werden die zu behandelnden Zellen auf einer porösen Membran dazwischen eingesetzt.

Die Erfindung Wird im folgenden näher unter Bezugnahme auf die Zeichnung erläutert die vorteilhafte Ausführungsbeispiele schematisch veranschaulicht Es zeigen:
- Fig. 1: eine Prinzipdarstellung,
- Fig. 2: eine Vorrichtung mit eingeschliffenem Deckel,
- Fig. 3: eine Vorrichtung mit elastisch dichtendem Deckel,
- Fig. 4: eine Vorrichtung mit Schraubdeckel,
- Fig. 5: eine Vorrichtung mit Rastdeckel
- Fig. 6 - 8: unterschiedliche Ausführungsformen mit kompartimentierter Vorrichtung,
- Fig. 9: einen Teilschnitt, der eine Abdichtungsmöglichkeit veranschaulicht,
- Fig. 10: eine Vorrichtung mit Elektrode und
- Fig. 11: eine weitere kompartimentierte Vorrichtung.

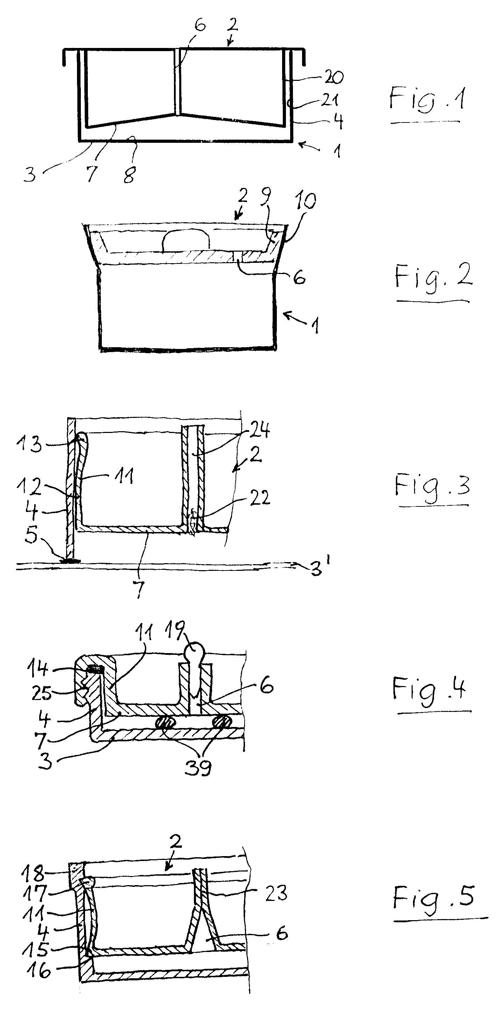

Die Vorrichtung besteht grundsätzlich aus dem Gefäß 1, beispielsweise einer Petrischale und einem Deckel 2. Das Gefäß 1 hat eine Bodenplatte 3 und Wände 4. Diese können einstückig ausgeführt sein oder auch mehrteilig. In Fig. 3 ist angedeutet, daß die Bodenplatte von einem Objektträger 3' gebildet ist, an dem die Wände 4 in bekannter Weise mittels eines lösbaren Klebers 5 befestigt sind. Statt dessen kann auch ein Dichtungsring in Verbindung mit einer Einrichtung zum Halten der Wände unter Einschluß des Dichtungsrings am Objektträger verwendet werden. Gefäß und Deckel bestehen aus einem Werkstoff, der das Substrat und den ggf. durchzuführenden Prozeß nicht negativ beeinflußt, beispielsweise Glas oder geeignetem Kunststoff. Es kann sich um Einzelgefäße handeln; alternativ sind mehrere Gefäße als Gruppe einstückig zusammenhängend vorgesehen.

In manchen Arbeitsphasen mag die Schale 1 ohne den besonderen Deckel 2 verwendet werden, beispielsweise offen oder mit einem Auflegedeckel. Will man die in der Schale befindlichen Zellen molekularbiologisch oder gentechnisch behandeln oder untersuchen, kann man statt dessen den erfindungsgemäßen Deckel 2 verwenden, der die Schale dicht an den Wänden 4 abschließt. Er besitzt eine Öffnung 6, die in den Ansprüchen als Abströmöffnung bezeichnet wird, weil durch sie die Atmosphäre abströmen kann, die beim Einsetzen des Deckels in das Gefäß verdrängt wird. Damit die Luft restfrei entfernt wird, ist es dabei im allgemeinen auch erforderlich, einen Teil der Flüssigkeit zu verdrängen. Die Abströmöffnung ist daher so dimensioniert, daß dies möglich ist. Der Abstand zwischen der Unterfläche der Deckelplatte 7 und der Oberseite 8 des Bodens beträgt zur Beobachtung in der Regel 20 µm, kann aber je nach Bedarf auch wesentlich größer oder kleiner sein. Die Abströmöffnung kann bei Bedarf nach Aufsetzen des Deckels verschlossen werden.

Die jeweils gewählte Art der Abdichtung des Deckels 2 gegenüber den Wänden 4 hängt vom gewählten Material ab. Wird Glas verwendet, kann man sie mittels Schliff erreichen. Dies ist in Fig. 2 veranschaulicht. Der Deckel 2 und das Gefäß 1 sind mit übereinstimmend und komplementär geschliffenen Kragen 9, 10 versehen. Die Abströmöffnung 6 befindet sich an geeigneter Stelle innerhalb des vom Kragen umgebenen Deckelbereichs. Eine solche Form des Gefäßes und des Deckels kann auch bei anderen Werkstoffen als Glas verwendet werden.

Wird für die Gefäßwände und/oder den Deckel elastisch nachgiebiges Kunststoffmaterial verwendet, kann man die Dichtung durch federnde Anpressung einer Dichtkante erreichen, wie dies in Fig. 3 angedeutet ist. Die Wände 4 des z. B. aus Glas oder Kunststoff bestehenden Gefäßes verlaufen parallel zueinander und lotrecht zum Boden 8, beispielsweise zylindrisch. Der Deckel 2 besteht aus einem elastischen Kunststoff. Von seiner vorzugsweise ebenen Bodenplatte 7 springt ein Kragen 11 auf, der im unteren Bereich 12 etwa parallel zu den Wänden 4 verläuft und mit diesem kein oder nur ein geringes Spiel einschließt. Der obere Rand 13 des Kragens 11 hat einen geringfügig größeren Durchmesser, so daß er beim Einsetzen in die Wand 4 elastisch zusammengedrückt wird und dadurch über seinen gesamten Umfang mit Vorspannung und dicht an der Innenfläche der Wand 4 anliegt. Die Stellung des Deckels im Gefäß wird durch seine Reibung an den Gefäßwänden gesichert.

Im Ausführungsbeispiel gemäß Fig. 4 schließt der Kragen 11 des Deckels ebenfalls mit nur geringer Spaltweite an die Wände 4 des Gefäßes an. Am freien Rand sind der Kragen 11 und die Wände 4 mit einem zusammenwirkenden Gewinde 25 versehen. Zwischen den Stirnflächen dieser Teile ist ein Dichtring 14 eingelegt. Auf den zwischen der Bodenplatte 3 und der Deckelplatte 7 eingelegten Ring 39 wird weiter unten eingegangen.

Statt des Schraubverschlusses kann auch ein irgendwie gearteter Schnappverschluß vorgesehen sein oder eine von außen auf den Deckel einwirkende, ihn in seiner Position haltende Klemme. Eine Ausführung mit Schnappverschluß ist in Fig. 5 dargestellt. Der Rand 15 der Deckelplatte 7 sitzt dichtend auf einem Absatz 16 der Wand 4 auf. In dieser Stellung wird er dadurch gehalten, daß der freie Rand 17 des Kragens 11 des Deckels 2 rastend mit einer Umfangsnase 18 am freien Rand der Gefäßwände zusammenwirkt. Der Absatz 16 hat auch den Vorteil, daß er einen Anschlag bildet, der den gewünschten Abstand zwischen dem Deckelboden 7 und dem Gefäßboden 3 sichert.

Die Deckelplatte 7 kann - wie in Fig. 1 gezeigt - so geformt sein, daß die Abströmöffnung 6 an erhöhter Stelle des Bodens beginnt. Dadurch kann die restlose Beseitigung von Luftblasen erleichtert werden. Im allgemeinen ist dies aber nicht erforderlich. Restliche Lufteinschlüsse können mit einem Überschuß des Kulturmediums bzw. der Reaktionsflüssigkeit herausgespült werden.

In den Beispielen gemäß Fig. 1, 3 und 4 ist vorgesehen, daß die Abströmöffnung 6 mit einem Steigrohr 24 verbunden ist, das mindestens ebenso hoch ist wie die zur Entlüftung des Spalts zwischen dem Deckelkragen 11 und den Gefäßwandungen 4 enthaltene Abströmstrecke. Durch den statischen Druck in dem Steigrohr kann gewünschtenfalls dafür gesorgt werden, daß Lufteinschlüsse aus dem Ringspalt zwischen der Umfangsfläche 20 des Deckels und der Innenfläche 21 der Gefäßwände vertrieben werden. Dieses Ziel kann man auch dadurch erreichen, daß man der Abströmöffnung einen so hohen Strömungswiderstand gegenüber Flüssigkeit verleiht und den Deckel so rasch einsetzt, daß der im Gefäß erzeugte Überdruck für die Verdrängung etwaiger Luft aus dem Ringspalt sorgt. Fig. 5 zeigt, daß sich die Abströmöffnung 6 nach oben hin zu einem Spalt 23 geringer Weite verengt, der als Drossel einen entsprechend hohen Strömungswiderstand hat. Dieser Spalt kann so ausgebildet sein, daß er sich in der Art eines Rückschlagventils beim Einsetzen des Deckels in das Gefäß unter elastischer Verformung des Materials für den Auslaß der Luft und von Überschußflüssigkeit öffnet, um sich anschließend unter der elastischen Rückstellkraft des Materials und/oder unter der Wirkung einer in der Zeichnung nicht dargestellten Klemme wieder luftdicht zu verschließen. Hingegen zeigt Fig. 4 für den Verschluß der Abströmöffnung einen Stopfen 19. Es besteht aber auch die Möglichkeit, die Öffnung mittels eines Öltröpfchens zu verschließen.

Wenn die Gefahr besteht, daß Zellen mit der verdrängten Flüssigkeit ausgeschwemmt werden, verschließt man die Abströmöffnung 6 mit einem Sieb 22, wie dies schematisch in Fig. 3 angedeutet ist.

Es können mehrere Öffnungen von der Art der Abströmöffnungen 6 vorgesehen werden, wenn man die Vorrichtung als Bioreaktor verwenden will, der von einem Reaktionsmedium durchströmt werden soll. In einem solchen Fall schließt man wenigstens eine Öffnung an einen Flüssigkeitszulauf und wenigstens eine andere Öffnung an einen Flüssigkeitsablauf an.

Die Anordnung einer Abströmöffnung innerhalb des vom Deckel eingenommenen Flächenbereichs hat den Vorteil, daß sie leicht verschlossen werden kann und daß die verdrängte Flüssigkeit an bestimmter Stelle austritt, an der sie beispielsweise mit einer Pipette abgenommen werden kann. Jedoch ist es auch möglich, die Abströmöffnung im Zwischenraum zwischen dem Deckelrand und den Wandungen des Gefäßes vorzusehen. Während oben vorausgesetzt war, daß der Deckelrand zum Zwecke der Führung oder Abdichtung sich eng an die Gefäßwände anschließt, wird zur Bildung eines Abströmquerschnitts an dieser Stelle ein hinreichender Abstand vorgesehen. Dieser kann auf dem ganzen Umfang oder auch nur an einer oder wenigen Stellen vorgesehen sein.

Die Anordnung der Abströmöffnung zwischen Deckelrand und Gefäßwänden schließt nicht aus, daß zwischen diesen ein dichter Abschluß geschaffen wird. Zum Beispiel kann im Ausführungsbeispiel der Fig. 3 auf das Abströmrohr 24 verzichtet werden, wenn im unteren Bereich 12 des Deckelkragens 11 ein hinreichender Abströmabstand zu den Gefäßwänden 4 vorgesehen wird. Beim Einsetzen des Deckels 2 in das Gefäß 1 kann durch diesen Umfangsabstand die eingeschlossene Luft und Überschußflüssigkeit abströmen. Erst wenn der Dichtrand 13 die Gefäßwände 4 erreicht, wird der Abströmvorgang gehemmt, aber nicht völlig ausgeschlossen, da sich der Dichtrand 13 je nach der Vorspannung, mit der er sich an die Gefäßwände 4 anlegt, unter dem inneren Überdruck in der Art eines Rückschlagventils hinreichend von den Gefäßwänden abheben kann, um ein weiteres Ausströmen zu ermöglichen. Erst wenn die die Einschubbewegung des Deckels veranlassende Kraft endet, legt er sich dicht an die Gefäßwände an.

Im Ausführungsbeispiel der Fig. 4 kann auf die Abströmöffnung 6 verzichtet werden, wenn der Abstand zwischen dem Deckelkragen 11 und den Gefäßwänden 4 sowie zwischen den zusammenwirkenden Gewindeflächen 25 groß genug ist, um das Abströmen zu gestatten. Dies endet erst dann, wenn der Deckel die gewünschte Endstellung erreicht und sich dabei der Dichtring 14 auf den Oberrand der Gefäßwände 4 auflegt.

In manchen Fällen kann gänzlich auf einen luftdichten Abschluß zwischen dem Deckelrand und den Gefäßwänden verzichtet werden, nämlich dann, wenn innerhalb einer die Vorrichtung aufnehmenden Apparatur für eine unschädliche Atmosphäre gesorgt wird und diese gegenüber der äußeren Atmosphäre durch einen entsprechenden Abschluß der Apparatur geschützt wird, der in diesem Fall den an der Vorrichtung selbst vorgesehenen Abschluß vertritt.

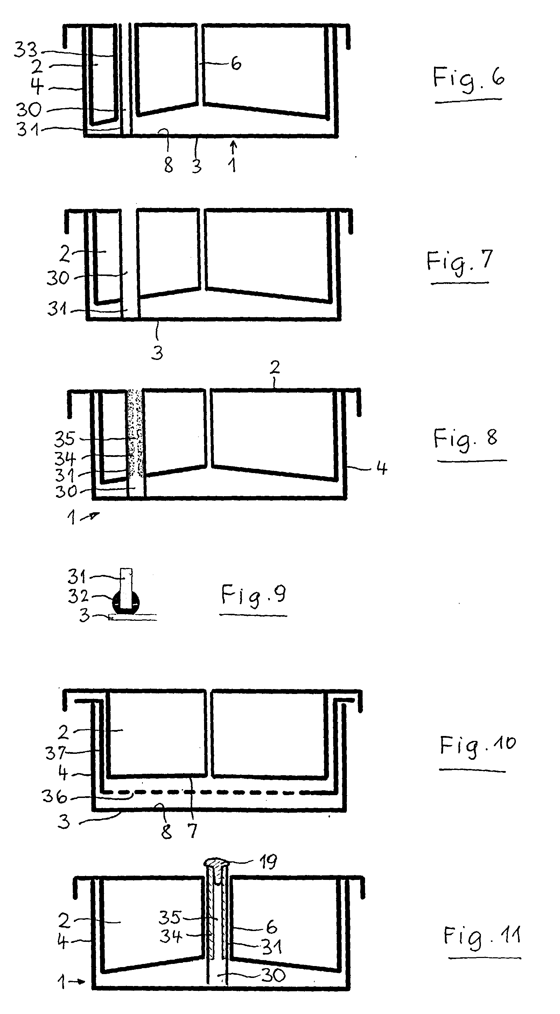

Die Ausführungsbeispiele gemäß Fig. 6 bis 8 basieren auf dem Ausführungsbeispiel gemäß Fig. 1, könnten aber auch ohne weiteres von irgendeiner anderen Ausführungsform der oben beschriebenen Erfindung ausgehen. Sie veranschaulichen die Ausbildung einer engeren Kammer 30 innerhalb eines weiteren Gefäßes 1. Die die Kammer 30 bildenden Wände 31, bei denen es sich um ein einfaches Rohr, beispielsweise aus Glas oder Kunststoff, handeln kann, sitzen in jedem Fall mit ihren unteren Enden auf der Oberseite 8 der Bodenplatte 3 auf, um die Kammer 30 von ihrer Umgebung zu trennen. Wenn die Wandung 31 nicht einstückig mit der Bodenplatte 3 ausgebildet ist, kann sie mittels einer geeigneten Dichtung daran angeschlossen sein. Die Dichtung kann durch ein Klebmittel oder gemäß dem in Fig. 9 dargestellten Beispiel durch einen auf den unteren Rand fest und dicht aufgesetzten Elastomerstreifen 32 gebildet sein. In manchen Fällen genügt es, wenn sich die Unterkante der Wandung 31 ohne zusätzliches Dichtmittel auf die Oberseite 8 des Bodens 3 aufsetzt. In diesem Fall sowie bei Verwendung einer nicht verklebten Elastomerdichtung ist es zweckmäßig, die dichte Anlage an der Bodenoberseite 8 dadurch zu fördern, daß die Wand 31 gegen den Boden 3 vorgespannt ist. Wenn sie gemäß Fig. 7 mit dem Deckel 2 aus einem Stück besteht, kann diese Vorspannung dadurch unter Nutzung der elastischen Eigenschaften des Gefäßes oder Deckels erzeugt werden, daß die Länge der Wandung 31 ein gewisses Übermaß gegenüber dem Bodenabstand des Deckels aufweist und der Deckel fest auf das Gefäß 1 aufgesetzt ist, beispielsweise mit Hilfe eines Schraub-, Reib- oder Schnappverschlusses. Eine solche Vorspannung kann aber auch dann erzeugt werden, wenn - wie im Ausführungsbeispiel gemäß Fig. 6 - die Wandung 31 lösbar in einer Aufnahme oder Halterung 33 des Deckels 2 gehalten ist, indem von der Halterung 33 eine entsprechende Reibkraft auf die Wandung 31 ausgeübt wird. Die Halterung 33 schließt die Wandung 31 zweckmäßigerweise dicht ein. Sie wird beispielsweise von einer Bohrung gebildet, in der die röhrenförmige Wandung 31 eng passend geführt ist. Diese Bohrung kann, wie Fig. 11 zeigt, von einer Abströmöffnung 6 gebildet sein.

Wenn der Deckel 2 und die Wandung 31 gesonderte Teile sind, müssen sie im Gefäß 1 genau zueinander positioniert sein. Dies erreicht man am leichtesten dadurch, daß die Wandung 31 zunächst in den Deckel 2 eingesetzt wird und dieser dann zusammen mit der Wandung in das Gefäß 1 eingesetzt wird. Oder es wird erst der Deckel aufgesetzt und dann die Wandung 31 durch den Deckel eingesetzt. Wenn es erforderlich ist, die Kammer 30 innerhalb des Gefäßes 1 einzurichten, bevor der Deckel 2 aufgesetzt wird, verwendet man am besten eine Positionierlehre für die Wandung 31, deren Abmessungen im Verhältnis zum Gefäß 1 denen des Deckels 2 gleichen. Diese Positionierlehre kann vor dem Einsetzen des Deckels 2 weggenommen werden, nachdem beispielsweise eine die Wandung 31 mit der Bodenoberseite 8 verbindende Klebung erhärtet ist, oder die Positionierlehre bleibt an Ort und Stelle unterhalb des Deckels 2, wenn dieser eingesetzt wird. Die Positionierlehre braucht im letzteren Fall kein gesondertes Teil zu sein; sie kann vielmehr konstruktiv mit der Wandung 31 vereinigt sein, indem diese beispielsweise eine Reihe von von ihr ringsum parallel zum Boden 3 des Gefäßes 1 oder zum Boden 7 des Deckels vorspringenden Armen aufweist, die in positionierender Anlage an der Innenfläche 21 der Gefäßwände 4 enden.

Ebenso wie das Gefäß 1 mit einem mit Abströmeinrichtung 6 ausgerüsteten Deckel 2 versehen ist, kann die Kammer 30 mit einem Verschlußorgan 34 versehen sein, das zur Begrenzung der Höhe des Substrats oberhalb des Gefäßbodens 8 geeignet ist und einen Abströmkanal 35 enthält, der wiederum mittels eines nicht gezeigten Stopfens verschließbar ist.

Ein weiteres Beispiel für Kompartimentierung ist in Fig. 4 veranschaulicht. Ein Dichtring 39 aus Elastomermaterial, beispielsweise ein handelsüblicher O-Ring, ist zwischen die Bodenplatte 3 und die Deckelplatte 7 eingelegt. Das Ringmaterial ist so dick, daß es in der Endstellung unter elastischer Deformation dicht an der Bodenplatte 3 und der Deckelplatte 7 anliegt. Dadurch wird innerhalb des Dichtrings eine Kammer geschaffen, die von dem außerhalb des Dichtrings gelegenen Bereich abgetrennt ist. Durch die Abströmöffnung 6 des Deckels oder irgendeine andere speziell zu diesem Zweck geschaffene Öffnung ist sie zugänglich, um eine andere Behandlung ihres Inhalts zu ermöglichen als im umliegenden Bereich. Der Ring 39 kann lose in das Gefäß 1 eingelegt werden, bevor der Deckel 2 aufgesetzt wird, wobei darauf geachtet werden muß, daß der von ihm umschlossene Bereich an der Stelle liegt, an der die Öffnung 6 zu erwarten ist. Damit die Kommunikation mit der Öffnung 6 gewährleistet ist und die Handhabung vereinfacht wird, kann der Ring auch mit dem Deckel 2 bleibend oder vorübergehend fest verbunden sein, beispielsweise durch eine Verklebung oder eine formschlüssige Halterung (in der Zeichnung nicht dargestellt).

Die erfindungsgemäße Kompartimentierung bietet die Möglichkeit, für einzelne oder mehrere Reaktionsschritte innerhalb der Kammer 30 etwas anderes zu tun als außerhalb derselben, wobei aber die thermischen Bedingungen dieselben bleiben. Auch können die Kulturen dieselben sein, wenn die Kammer 30 erst nach dem Anlegen der Kultur in dem Gefäß 1 eingesetzt wird. Beispielsweise kann in einem mehrschrittigen Verfahren in einem Schritt innerhalb der Kammer 30 doppelt soviel Enzym wie außerhalb der Kammer eingegeben werden. Danach wird die Kammerwand 31 wieder entfernt, und alle Zellen werden wieder identisch behandelt. Wenn mehrere derartige Einsetzkammern 30 in einem Gefäß verwendet werden, vermehren sich die Variationsmöglichkeiten entsprechend.

Sollen die Zellen in dem Gefäß einer Elektroporation oder Elektrofusion unterzogen werden, so werden im Deckel und Boden jeweils (in der Zeichnung nicht gezeigte) Metallelektroden vorgesehen. Die Zellen werden dann - wie in Fig. 10 gezeigt - auf einem porösen Boden 36 oder einer Membran eines Zellkultureinsatzes 37 zwischen Boden 3 und Deckel 2 eingebracht und einem elektrischen Feld ausgesetzt.

## Patentansprüche

1. Verfahren zum Erzeugen einer von der Atmosphäre abgeschlossenen Schicht einer eine Zellkultur enthaltenden Flüssigkeit zwischen einer Boden- und einer Deckelplatte, dadurch gekennzeichnet, daß ein die Deckelplatte aufweisender Deckel in ein von der Bodenplatte und davon hochstehenden Wänden gebildetes Gefäß unter Verdrängung der oberhalb der zu belassenden Schicht befindlichen Atmosphäre und Flüssigkeit eingesenkt und sein Rand dicht an die Wände angeschlossen wird.

2. Vorrichtung zur Aufnahme einer Zellkultur mit einem eine Bodenplatte (3) und davon hochstehende Wände (4, 31) aufweisenden Gefäß (1, 30) und einem Deckel (2, 34), insbesondere zur Durchführung des Verfahrens nach Anspruch 1, dadurch gekennzeichnet, daß der in das Gefäß (1, 30) einführbare Deckel (2, 34) mit einer Abströmöffnung (6, 35) versehen ist.

3. Vorrichtung nach Anspruch 2, dadurch gekennzeichnet, daß die Umfangsfläche (20) des Deckels (2, 34) sich im wesentlichen formgleich eng an die Innenfläche (21) der Wände (4, 31) anschließt.

4. Vorrichtung nach Anspruch 3, dadurch gekennzeichnet, daß die Umfangsfläche (20) des Deckels (2, 34) dicht mit der Innenfläche (21) der Wände (4, 31) zusammenwirkt.

5. Vorrichtung nach einem der Ansprüche 2 bis 4, dadurch gekennzeichnet, daß zwischen dem Rand (11) des Deckels (2) und dem Rand oder einem Absatz (16) der Gefäßwände (4) eine Dichtung vorgesehen ist.

6. Vorrichtung nach einem der Ansprüche 2 bis 5, dadurch gekennzeichnet, daß eine Halterung (13; 17, 18) zur Aufrechterhaltung der Dichtposition des Deckels (2) gegenüber dem Gefäß (1) vorgesehen ist.

7. Vorrichtung nach einem der Ansprüche 2 bis 6, dadurch gekennzeichnet, daß der Deckel (2) eine Deckelplatte (7) mit einer etwa parallel zur Bodenplatte (3) des Gefäßes (1) verlaufende Unterfläche aufweist.

8. Vorrichtung nach einem der Ansprüche 2 bis 7, dadurch gekennzeichnet, daß für die Abströmöffnung (6, 35) eine Einrichtung (19, 23) zum Abschluß von der Atmosphäre vorgesehen ist.

9. Vorrichtung nach einem der Ansprüche 2 bis 8, dadurch gekennzeichnet, daß die Abströmöffnung (6, 35) mit einem Sieb (22) versehen ist.

10. Vorrichtung nach einem der Ansprüche 2 bis 9, dadurch gekennzeichnet, daß die Bodenplatte (3) des Gefäßes (1) von einem Objektträger (3') gebildet ist, mit dem die Wände (4, 31) dicht verbunden sind.

11. Vorrichtung nach einem der Ansprüche 2 bis 10, dadurch gekennzeichnet, daß die Abströmöffnung (6) eine Steigstrecke (24) aufweist, deren Mündungshöhe mindestens etwa ebenso hoch ist wie das obere Ende des zwischen der Umfangsfläche (20) des Deckels (2) und den Gefäßwänden (4) befindlichen Spalts.

12. Vorrichtung nach Anspruch 2 oder einem der Ansprüche 5 bis 11, dadurch gekennzeichnet, daß die Abströmöffnung durch einen mindestens stellenweise vorgesehenen Abstand zwischen dem Deckelrand (11) und den Gefäßwänden (4) gebildet ist.

13. Vorrichtung nach Anspruch 12, dadurch gekennzeichnet, daß der Deckelrand einen von der unterseitigen Deckelplatte (7) hochstehenden Kragen (11) umfaßt, dessen oberer Rand (13, 14) mit den Gefäßwänden (4) zur Bildung einer Dichtung zusammenwirkt.

14. Vorrichtung nach einem der Ansprüche 2 bis 13, dadurch gekennzeichnet, daß eine Einrichtung (31) zum Abteilen mehrerer voneinander getrennter Kammern innerhalb des Gefäßes (1) vorgesehen ist.

15. Vorrichtung nach einem der Ansprüche 2 bis 14, dadurch gekennzeichnet, daß ein engeres Gefäß (30) zur Unterbringung in einem weiteren Gefäß (1) vorgesehen ist und der Deckel (2) des weiteren Gefäßes lediglich zum Verschluß des außerhalb des engeren Gefäßes (30) befindlichen Bereichs des weiteren Gefäßes (1) ausgebildet ist.

16. Vorrichtung nach Anspruch 15, dadurch gekennzeichnet, daß der Deckel (2) einen dem Umfang des engeren Gefäßes (30) angepaßten Ausschnitt (33) aufweist.

17. Vorrichtung nach Anspruch 16, dadurch gekennzeichnet, daß der Ausschnitt (33) von der Abströmöffnung (6) gebildet ist.

18. Vorrichtung nach einem der Ansprüche 15 bis 17, dadurch gekennzeichnet, daß der Deckel (2) als Halterung (33) für das engere Gefäß (30) ausgebildet ist.

19. Vorrichtung nach Anspruch 18, dadurch gekennzeichnet, daß der Deckel (2) mit dem engeren Gefäß (30) zu gemeinsamer Handhabung verbunden ist.

20. Vorrichtung nach Anspruch 15, dadurch gekennzeichnet, daß für das engere Gefäß (30) ein von dem Deckel (2) des weiteren Gefäßes (1) gesonderter Deckel (34, 19) vorgesehen ist.

21. Vorrichtung nach Anspruch 15, dadurch gekennzeichnet, daß ein sowohl das weitere Gefäß (1) als auch das engere Gefäß (30) verschließender Deckel vorgesehen ist.

22. Vorrichtung nach einem der Ansprüche 15 bis 21, dadurch gekennzeichnet, daß zwischen Bodenplatte (3) und Deckelplatte (7) ein eine gesonderte Kammer einschließender Ring (39) angeordnet ist und der Deckel im Bereich dieser Kammer eine Öffnung (6) aufweist.

23. Vorrichtung nach einem der Ansprüche 2 bis 22, dadurch gekennzeichnet, daß wenigstens ein Zellkultureinsatz (37) vorgesehen ist.
